# EUROPEAN PATENT APPLICATION

(11) **EP 2 371 940 A1**
(43) Date of publication of application: **05.10.2011**
(21) Application number: 10158619.6
(22) Date of filing: 31.03.2010
(51) Int. Cl.: C12M 1/00

(54) **Process for bio-oil production involving the use of CO2**

(71) Applicant: B.T.Biochemical Tissues S.R.L., 09028 Sestu (IT)
(72) Inventor: Cao, Giacomo, 09129, CAGLIARI (IT); Concas, Alessandro, 09100, CAGLIARI (IT)
(74) Representative: Cattaneo, Elisabetta

(57) **Abstract**

A process for the production of bio-oil involving the use CO₂, and the related plant for its implementation are described. In particular, the invention concerns a process for the production of bio-oil and for the contextual reduction of CO₂ from punctual sources of emission, the process including a step wherein said gas passes through a solid support and, through the same support, simultaneously passes sea and/or fresh water for subtracting CO₂ to the gas. Sea and/or fresh water enriched in CO₂ and integrated with macro-nutrients is used to feed an algal culture in photobioreactor, wherein the algae grow and propagate. From algal biomass is then extracted the bio-oil.

## Description

### FIELD OF THE INVENTION

The present invention concerns a process for producing bio-oil, involving the use of CO₂, and a plant for the implementation thereof.

Particularly, said process is based on the use of algae from which the bio-oil is extracted, once said algae have been grown and propagated by photosynthesis. The process of the present invention allows to prepare biofuels from bio-oil, such as biodiesel, green coal and residual cakes.

Moreover, besides bio-oil, compounds useful as raw material in industry, such as food, biomedical, cosmetic and zoo-technical industry, can be contextually obtained.

### STATE OF THE ART

In developed countries, about 70% of oil is employed by the transportation sector. Since this sector absorbs about 30% of the entire national energetic needs, transportation sector represents the main item of oil consumption. In fact, electrical energy may be generated by using coal or natural gas as well as by exploiting nuclear, hydroelectric, energy.

Nowadays, the rise of oil price and the climate change concerns deriving from the use of fossil fuels, are becoming urgent issues that need to be solved in order to ensure a worldwide sustainable development.

As a result, in order to reduce the use of fossil fuels, alternative fuels to the "oil-derived" ones, are currently being investigated by scientific e technologists community.

The exploitation of biofuels for replacing fossil fuels in the transportation sector, has been already proposed.

Biofuels derive from biological sources and in particular from plants, therefore being produced mainly through photosynthetic mechanisms.

Recently the use of algae, as vegetal source for producing bio-oil, by promoting photosynthetic mechanisms within suitable photobioreactors, has been proposed.

This choice is based upon the observation that intrinsic properties of microalgae can lead to great advantages when they are used for producing biofuels. In fact, microalgae can be grown in industrial sites as well as in arid zones thus avoiding to subtract land to agriculture. Moreover, high growth rate and high oil content of algae, determine a higher areal productivity (up to three orders of magnitude in some cases) when compared with the one obtainable by using typical crops for first generation biofuels.

The analysis of scientific literature and of patents shows that, in the field of microalgae, the main focus of technologists and researchers is the optimization of photobioreactors' productivity. In particular, optimization of light distribution within the culture was the main target of researchers and technologists. Photobioreactors have been already used to produce large quantities of algal biomass to be used as raw material in different fields from the energetic one (Molina Grima, E., Acién Fernández, F.G., García Camacho, F. and Chisti Y. "Photobioreactors: light regime, mass transfer, and scale-up", Journal of Biotechnology, 70, 231-47 (1999); Tredici, M.R. Bioreactors, photo. In: Flickinger MC, Drew SW, editors. "Encyclopedia of bioprocess technology: fermentation, biocatalysis and bioseparation", Wiley, 395-419, (1999); Molina, G., Acién, Fernández, F.G., García Camacho, F., Camacho Rubio, F. and Chisti, Y. "Scale-up of tubular photobioreactors", Journal of Applied Phycology;12, 355-68, (2000); Pulz, O. "Photobioreactors: production systems for phototrophic microorganisms", Applied Microbiology and Biotechnology, 57, 287-93 (2001); Carvalho, A. P., Meireles. L. A., Malcata, F. X. "Microalgal reactors: a review of enclosed system designs and performances", Biotechnology Progress, 22, 1490-506 (2006)).

In the paper by Chisti, (Chisti Y. "Biodiesel from microalgae beats bioethanol", Trends in Biotechnology 26,126 (2008), a process for producing microalgal biomass is proposed. The process is based upon the supply of carbon dioxide from flue gas, water and inorganic nutrients to a microalgal culture. Bio-oil is then extracted from microagal biomass and thus converted in biodiesel. Solid byproducts, resulting from bio-oil extraction process, are then sent to an anaerobic reactor for the production of biogas. Most of the electric energy produced from biogas combustion, is used for the energetic self-sustainment of the process, while the biodiesel is sold in the fuels market. In the paper by Chisti the process is defined only on a conceptual level. In fact no sizing of plant units or quantification of flows is performed. This fact do not allows a real evaluation of the technical and economical feasibility of the process.

Another process based upon microalgae for the production of biodiesel is proposed in the U.S. patent US2008/0160593. In this process, atmospheric carbon dioxide is used in order to ensure microalgae growth. As reported at the paragraph [0054] of the patent, very extended areas are needed for guaranteeing a reasonable productivity of the proposed process.

A further process based on the use of microalgae for the production of biodiesel is proposed in the international patent PCT/US2007/015514 by Greenfuel Technologies Corporation. In this patent a process for the treatment of oil sand is proposed. The treatment of oil sands produces CO₂ as by product. According to the patent, CO₂ is sent to a photobioreactors' system where phototrophic microorganisms use it to grow and replicate. From the obtained microalgal biomass it is possible to extract bio-oil which, in turn, can be further processed for producing biodiesel. The process is limited to the treatment of oil sand and the use of microalgae has to be considered only as an ancillary step that is aimed to recycle a gaseous by product such as CO₂.

Further "integrated processes" for the production of biofuels from algae have been proposed. The expression "integrated process" refers to a procedure where remediation and bio-oil production are realized simultaneously. In these processes the pollutants may be seen as raw material from which bio-oil is produced.

An example of "integrated process" for production of biofuels is proposed in the international patent application PCT/US2007/081141 by General Atomics. This process involves a scrubber, where a gas stream contaminated by carbon dioxide, sulphur oxides and nitrogen oxides moves upward through a chamber where a liquid solution of sodium hydroxide and sodium bicarbonate is sprayed downward producing a countercurrent flow.

The outlet liquid flow from the scrubber is then rich in nutrients such as nitrogen and phosphor, sulphur etc. This liquid is then fed in a bioreactor where it acts as culture broth wherein microalgae can grow. Although the process is very interesting on a conceptual level, reaction yields are not specified in the patent neither quantitatively nor qualitatively. Moreover the use of synthetic compounds such as sodium hydroxide, sodium bicarbonate and nutrients may determine an increase of plant operating costs thus affecting economic feasibility of the process. In view of what above reported with reference to the scientific and patent literature on the matter, it is an object of the present invention to provide process for producing bio-oil showing high efficiency and allowing to overcome the drawbacks above mentioned with reference to the known processes.

### SUMMARY OF THE INVENTION

The above mentioned object has been achieved by a process for the production of bio-oil comprising the steps of:
a1) providing a gas having high CO₂ content;
a2) providing sea and/or fresh water for the production of the culture medium;
b1) splitting the stream of said gas into two flows q1 and q2;
b2) passing the gas flow q1, having high CO₂ content, through a solid support;
b4) passing the sea and/or fresh water through the same solid support of step b2) in order to subtract CO₂ from said gas, said step b4) being simultaneously performed with step b2);
c) adding nitrogen-rich and/or phosphorus-rich nutrients to the sea and/or fresh water, previously enriched in CO₂, to form a nutrient mixture;
d1) contacting said nutrient mixture with an algal culture;
d2) contacting said nutrient mixture and said algal culture with the gas flow q2;
e) exposing said algal culture, contacted with the nutrient mixture and with the gas flow q2, to a light source capable to promote photosynthesis, thus obtaining the formation of algal biomass;
f) separating the obtained algal biomass; and
g) extracting bio-oil from the algal biomass.

In another aspect, the invention concerns a plant 1 for the implementation of the process of the invention, comprising:
- at least one unit 3 for splitting the gas stream into two gas flows q1 and q2;
- at least one absorption unit 4 for the transfer of CO₂ from gas flow q1, having high content of CO₂ in sea and/or fresh water, this unit including at least one column wherein a solid support is packed;
- at least one mixing unit 5 of said sea and/or fresh water enriched in CO₂ and nutrients rich in nitrogen and phosphorus;
- at least a photobioreactor 6 for the production of algal biomass, wherein an algal culture contacts the outlet mixture from said mixing unit 5 and the gas flow q2 having a high CO₂ content;
- at least one unit 7 for separating the biomass from the fluid outgoing from the at least one bioreactor 6; and
- at least one unit 8 for extracting the bio-oil from the separated biomass.

According to the present invention, the gas having high CO₂ content, preferably comprises exhaust fumes from thermal power stations or industrial plants.

In the present invention, when using:
- "gas having a high CO₂ content", it is meant a gas having a minimum CO₂ content not lower than the CO₂ content of the atmosphere;
- "algal culture", it is meant a culture of algal cells including the culture broth and the nutrients required for developing photosynthetic activity;
- "algal biomass", it is meant the algal culture enriched with the products of photosynthesis deriving from the process of invention.

### BRIEF DESCRIPTION OF THE DRAWING

The features and advantages of the invention will be apparent from the following detailed description, from the working example provided for illustrative and non limiting purposes, and from the attached Figure wherein a flow-sheet of the process for producing bio-oil is shown according to the Example of the present invention.

### DETAILED DESCRIPTION OF THE INVENTION

The subject of the invention is therefore a process for the production of bio-oil which comprises a step of pre-treatment of a gas having a high CO₂ content. In particular, the invention concerns a process for the production of bio-oil comprising the steps of:
a1) providing a gas having high CO₂ content;
a2) providing sea and/or fresh water for the production of the culture medium;
b1) splitting the stream of said gas into two flows q1 and q2;
b2) passing the gas flow q1, having high CO₂ content, through a solid support;
b4) passing the sea and/or fresh water through the same solid support of step b2) in order to subtract CO₂ from said gas, said step b4) being simultaneously performed with step b2);
c) adding nitrogen-rich and/or phosphorus-rich nutrients to the sea and/or fresh water, previously enriched in CO₂, to form a nutrient mixture;
d1) contacting said nutrient mixture with an algal culture;
d2) contacting said nutrient mixture and said algal culture with the gas flow q2;
e) exposing said algal culture, contacted with the nutrient mixture and with the gas flow q2, to a light source capable to promote photosynthesis, thus obtaining the formation of algal biomass;
f) separating the obtained algal biomass; and
g) extracting bio-oil from the algal biomass.

The process of the invention thus involves that, in step a1), a gas having a high CO₂ content is provided. According to the invention, the higher the concentration of CO₂ in gas, the higher the algal growth rate and, consequently, the greater the process productivity and effectiveness.

Preferably, the gas provided in step a1) comprises exhaust fumes from thermal power stations or industrial plants. In this way, the process, besides showing high yields in terms of algal growth and bio-oil production, contextually allows to advantageously reduce CO₂ emissions.

More preferably, the provided gas consists of exhaust fumes from thermal power stations or industrial plants. In fact, it has been resulted extremely convenient both for the implementing purposes of the process and for the reduction of air pollution to directly and entirely receive the exhaust fumes from power stations or industrial installations.

According to a preferred embodiment, the gas is cooled before being provided in step a1).

As shown herein below, in step a2), sea and/or fresh water is provided, that is used for the production of the culture medium.

In step b1), the gas stream is divided into two flows referred to as q1 and q2 respectively.

In step b2) of the process, the gas flow q1 of step b1) passes through a solid support.

According to a preferred embodiment, the process further comprises a step b3), to be performed after step b2) and before step b4), wherein said sea and/or fresh water is mixed with NaOH. In fact, NaOH, besides limiting the acidity increase resulting from absorption of CO₂ in the liquid phase, reacts with CO₂, which is then consumed according to the following general stoichiometry:

4NaOH + 2CO₂ → 2Na₂CO₃ + 2H₂O

This reaction reduces the CO₂ concentration in the liquid phase thus maintaining always a high concentration gradient between CO₂ in gas phase and the unreacted CO₂ absorbed in liquid phase. This, in turn, results in an increase of the driving force underlying the CO₂ transfer from the gas phase to the liquid one, thus in an increase of the CO₂ absorption capacity of liquid phase. Consequently, as better shown herein below for the plant, being equal the length of columns where the solid support is placed, it is advantageously possible to increase the CO₂ amount that is subtracted to the gas phase.

Moreover, the above described reaction determines the formation of a compound (Na₂CO₃) which is easily metabolized by the algae and thus ensures a continuous supply of nutrients important for algae growth.

In step b4), sea and/or fresh water, preferably mixed with NaOH, simultaneously passes through said solid support in order to subtract CO₂ to the gas.

As it will be apparent from the description of the plant according to the present invention, this step is advantageously carried out by using a solid support, preferably made of ceramic or metal material, through which the gas having a high CO₂ content passes simultaneously with sea and/or fresh water.

In the contact areas between liquid (sea and/or fresh water) and gas, transfer of carbon dioxide takes place, by molecular diffusion, from the gas toward the liquid phase. In this way, sea and/or fresh water enriched in CO₂ is obtained.

The possibility of using sea water as a liquid for CO₂ removal involves the following advantages. First, the seawater is a costless raw material which is available in huge amounts without the environmental and logistical drawbacks that may occur, for example, when groundwater or river water are used.

Moreover seawater, and in particular Mediterranean water, is characterized by concentration levels of micro-elements, such as Si, Fe, Mg, Ca etc., dissolved therein so as to ensure microalgal growth and propagation. This results in a significant reduction in operating costs, since the cost items related to the purchase of such micro-elements are no longer present.

Moreover alkalinity of seawater and the presence of salts dissolved therein respectively allow to limit the acidity of solution resulting from the absorption and to increase the amounts of CO₂ transferrable in step b4) in view of the CO₂ consuming reactions that can take place in the liquid phase.

In case the gas deriving from power stations or industrial plants is used, a further advantage is achieved since these plants are usually located near to the sea, in order to ensure the water needed for cooling processes. This results in the reduction of costs associated to water pumping, significant simplifications as well as saving of costs related to the implementation of pipelines.

Alternatively, the process of the invention can also use fresh water, as such or mixed with sea water. In case fresh water as such is used, it should be verified that micronutrients' concentration is so as to ensure the growth of algal biomass, otherwise micronutrients should be integrated.

According to the preferred embodiment that uses the exhaust fumes from power or industrial plants, the average values of the chemical-physical characteristics of water, that influence carbon dioxide transfer from fumes to sea and/or fresh water, are preferably as shown Table 1. It should be noted that values in Table 1 have been reported with reference to the water of Mediterranean Sea, therefore variations, albeit minimal, are not excluded when seawater from a different sea is employed.

**Table 1**

| **Property** | **U.M.** | **Value** |
|---|---|---|
| Carbon dioxide diffusivity in gas phase × 10³ | cm² s⁻¹ | 169.00 |
| Carbon dioxide diffusivity in liquid phase × 10⁶ | cm² s⁻¹ | 21.06 |
| Surface tension of the liquid phase | dyn cm⁻¹ | 30.00 |
| Viscosity of liquid phase | cp | 1.06 |
| Viscosity of gas phase | cp | 0.018 |
| Head loss in packed bed | bar | 0.169 |
| Linear head loss in packed bed | bar m⁻¹ | 0.022 |

Therefore from step b4), sea and/or fresh water enriched of Na₂CO₃ and carbon dioxide is obtained, while the outlet gas is substantially depleted of CO₂ and thus can be discharged in the atmosphere.

Advantageously, in the preferred embodiment, which employs exhaust fumes from a power station, steps b2) and b4) can advantageously also lead to a significant removal of pollutants such as NOₓ and SOₓ, from the exhaust gas.

Preferably, the direction along which the gas having high CO₂ content of the step b2) passes through the solid support is opposite to the direction along which the sea and/or fresh water, preferably mixed with NaOH, of the step b4) passes through the same support. In fact, the choice of passing the gas in counter-current respect to sea and/or fresh water results in a more efficient transfer of CO₂ from the gas phase to the liquid one.

The outletting liquid from step b4) therefore consists of sea and/or fresh water containing CO₂, preferably Na₂CO₃, and micro-nutrients that are necessary for the photosynthetic growth of microalgae.

However, the concentration of macro-nutrients such as nitrogen and/or phosphorus, is not sufficient to ensure the algal growth. The amount of additional macro-nutrients can be estimated by means of simplified stoichiometric reactions reported in the literature, such as the following:

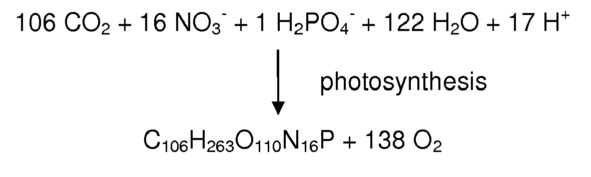

Therefore, according to the present invention, macronutrients (nitrogen and/or phosphorus) are provided in step c) to the sea and/or fresh water, enriched in CO₂, in order to form a nutrient mixture.

Preferably, these macro-nutrients are provided as wastewater. In this way, a double advantage may be achieved. On the one hand, the recycling of wastewater determines an environmental benefit, on the other hand, the availability of costless nutrients translates to economical benefit. In fact, otherwise macro-nutrients have to be purchased *ad hoc,* thus adding a not negligible cost item to the process. More preferably, said wastewaters are purified, not denitrified, not dephosphorized, and optionally pasteurized.

In fact, purified but non denitrified or non dephosphorized wastewaters have nitrogen and phosphorous concentrations such as, even added in small amounts to the CO₂-enriched seawater, to obtain a mixture containing nitrogen and phosphorus concentration allowing to achieve a good algal growth. Advantageously, the process of the invention presents a step of pasteurisation of wastewaters, that is performed previous to their use in step c), in order to employ even wastewaters with high bacterial charges.

The amount of wastewater to be added can be calculated on the base of the concentration of macro-nutrients already present in the sea and/or fresh water from step b4) and the concentration of the same macro-nutrients in the wastewater.

The step d1) of the present invention provides that the nutrient mixture of step c), with sea and/or fresh water enriched in CO₂, and preferably in Na₂CO₃, nutrients rich in nitrogen and/or phosphorus, contacts an algal culture.

The choice to employ an algal culture, preferably of microalgae, is based upon their capability to use the carbon dioxide and the light for surviving and propagating by exploiting photosynthesis.

These features of algae conveniently allow, being equal the areas involved, much higher productivity (also three orders of magnitude in some cases) compared to those of plants grown on land. Furthermore, their growing can advantageously take place in industrial or arid areas hence without subtracting useful areas to agriculture for food production, thus avoiding the competitiveness concerns of "food for fuel".

An important peculiar characteristic of the microalgae is to have very high propagation rate and bio-oil content, which can be extracted from, ranging from 40 to 70% of total cell weight.

The step d2) provides that the algal culture and nutrients' mixture above described contact the gas flow q2 CO₂-enriched.

The algal culture, so contacted with the said nutrients' mixture, is exposed to a light source capable to promote photosynthesis, thus allowing the formation of algal biomass, according to the step e) of the present invention. Preferably, the light source is a natural source, i.e. sunlight, even if artificial light can be used when sunlight is not sufficient for promoting photosynthesis.

Once formed, algal biomass is separated from fluids in the step f). Preferably, such a separation is carried out by sedimentation or filtration.

According to a preferred embodiment, fluids separated from biomass can pass again through the support of steps b2)-b4) and therefore be conveniently and advantageously recycled.

Moreover, once the process starts, part of algal biomass is advantageously recirculated and hence re-used in step d1), i.e. re-contacts the nutrients' mixture to form further biomass, with great advantages in terms of economical feasibility and efficiency. The amount of biomass to be recycled depends on the specific photobioreactor considered and the biomass concentration coming out from the same.

Preferably, the process of the present invention further comprises a step h) of drying of the algal biomass subsequent to the separation step f). The drying step is aimed to remove residual humidity from algal biomass after its separation from fluids.

According to a preferred embodiment, the gas is cooled before being provided in step a) and the heat so recovered is employed in step h) for drying microalgal biomass, thus achieving a clearly advantageous energy saving.

Finally, the step g) is aimed to extract bio-oil from algal biomass. Preferably, extraction is carried out by mechanical pressing of biomass, mainly for economical reasons.

From the extraction, a bio-oil having a high triglycerides content and a residual solid cake are obtained.

The obtained bio-oil as such is not suitable to be used as biofuel, especially in fast diesel engines, due to its high viscosity (70-80 CST at 20°C against 4-7 pm CST diesel). A clear improvement of this feature can be obtained by means of transesterification reaction which determines the rupture of triglycerides' molecule thus generating a less viscous liquid. The transesterification reaction may involve the use of methanol and a catalyst such as sodium hydroxide. The products of this reaction are biodiesel and glycerine which are separated by suitable systems such as for example by settling.

As far as the residual solid cake is concerned, different industrial applications are possible depending on its compositional characteristics. In fact, depending on the type of algal strain used (e.g., Porphyridium cruentum, Thalassiosira pseudonana, Chlorella vulgaris), a residual solid cake containing high concentrations of high added value compounds may be obtained, such as vitamins and antioxidants (e.g. β-carotene and astaxanthin), hormones precursors (sterols), that allow the recycling of solid cake in the agro-food, pharmaceutical and cosmetic industry (Rodriguez-Garcia, I., Guil-Guerrero, J. L. Evaluation of the antioxidant activity of three microalgal species for use as dietary supplements and in the preservation of foods. Food Chemistry 108, 1023-1026 (2008)). When specific algal strains are used (eg *Spirulina platensis, Rhodella reticolata, Porphyridium sp., Porphyridium cruentum, Paeodactylum tricornutum, Tetraselmis suecica, Chlorella autotrophica, Dunaliella tertiolecta, Dunaliella bardawil, Isochrysis galbana, Isochrysis galbana 6ar Tiso, Ellipsoidon sp., Tetraselmis tetrathele*) the residual solid cake may be used in the biomedical industry as antimicrobial, antiviral and anticancer compounds (Bing Li, B., Zhanga, X., Gao, M., and Chu, X. Effects of CD59 on antitumoral activities of phycocyanin from Spirulina platensis. Biomedecine & Pharmacotherapy. 59, 551-560 (2005); Yasumoto, T., Satake, M. Bioactive Compounds from Marine Microalgae. CHIMIA International Journal for Chemistry, 52, 63-68 (1998); Talyshinsky, M., Souprun, Y., Huleihel, M.Anti-viral activity of red microalgal polysaccharides against retroviruses. Cancer Cell International, Published online. doi: 10.1186/1475-2867-2-8 (2002); Fabregas, J., Garcia, D., Fernandez-Alonso, M., Rocha, A.I.,Gomez-Puertas, P., Escribano, J.M., , Otero, A., Coll, J.M. In vitro inhibition of the replication of haemorrhagic septicaemia virus (VHSV) and African swine fever virus (ASFV) by extracts from marine microalgae. Antiviral Research 44, 67-73 (1999)).

Alternatively, in case the compositional characteristics do not allow the above described applications, the residual solid cake can be used as fuel. In fact, it can be packed into pellets to be burned as "green coal" in thermal power stations, thus leading to significant energy savings.

In another aspect, the present invention relates to a plant 1 for producing bio-oil. In particular, this plant 1 comprises:
- at least one unit 3 for splitting the gas stream into two gas flows q1 and q2;
- at least one absorption unit 4 for the transfer of CO₂ from gas flow q1, having high content of CO₂ in sea and/or fresh water, this unit including at least one column wherein a solid support is packed;
- at least one mixing unit 5 of said sea and/or fresh water enriched in CO₂ and nutrients rich in nitrogen and phosphorus;
- at least a photobioreactor 6 for the production of algal biomass, wherein an algal culture contacts the outletting mixture from said mixing unit 5 and the gas flow q2 having a high CO₂ content;
- at least one unit 7 for separating the biomass from the fluid outgoing from the at least one bioreactor 6; and
- at least one unit 8 for extracting the bio-oil from the separated biomass.

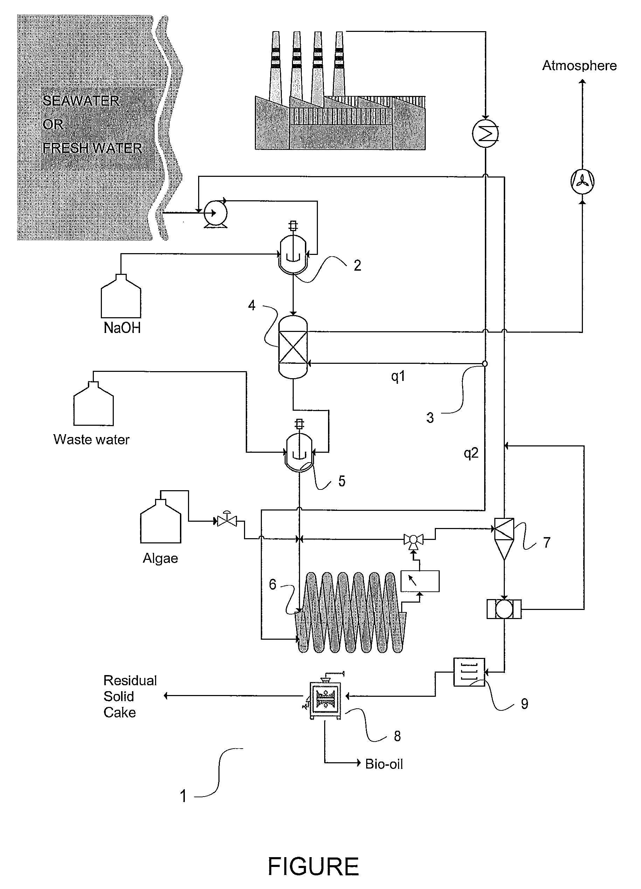

With reference to Figure 1, which represents the plant 1 of the invention, sea and/or fresh water is provided to the at least one absorption unit 4. According to a preferred embodiment, the plant 1 further comprises at least one mixing unit 2 where sea and/or fresh water and NaOH are fed and mixed in order to form a mixture called "absorbent liquid" which, in turn, is fed to the at least one absorption unit 4.

In the at least one splitting unit 3 the gas stream from the emission source is split into two flows, q1 and q2, the first one feeds the absorption unit 4, while the second is sent to the photobioreactor unit 6 as explained below.

In the at least one absorption unit 4, the inletting gas flow q1 passes through a solid support that is packed within the at least one column. The absorbent liquid simultaneously passes through said at least one column, thus subtracting CO₂ to the gas as described in the steps b) of the process of the invention. Thus, the outletting absorbent liquid from the at least one absorption column is enriched in CO₂, and preferably also in Na₂CO₃, while the gas is correspondingly depleted of CO₂.

Preferably, in the at least one column, the gas flows along a direction that is opposite to the one along which the absorbent liquid passes. This results in a more efficient transfer of CO₂ from the gas phase to the liquid phase.

With reference to Figure 1, according to a preferred embodiment, the gas flows upward through said column while the absorbent liquid leaches downward through said column by exploiting only gravity. This advantageously simplifies the realization of the plant 1.

Preferably, the solid support within the at least one column of the at least one absorption unit 4 is preferably made of ceramic or metal material, since are materials which allow an advantageous high absorption of gas.

More preferably, said solid support in the at least one column of the at least one absorption column of the at least one absorption unit 4 is in the form of Raschig rings, Berl saddles, Intalox saddles, and Lessing rings, Pall rings, spiral rings, cross rings or combinations thereof. As will be apparent from the following example, these solid supports, once packed within the column, show a high surface area that advantageously promote a high contact area between the passing through gas and seawater, thus enhancing the transfer of CO₂. Advantageously, in the preferred embodiment employing the exhaust fumes from thermal power stations, the absorption unit 4 also allows to significantly abate residual pollutants present in the exhaust fumes, such as NOₓ and SOₓ.

In the at least one mixing unit 5, the absorbent liquid enriched in CO₂, and preferably also in Na₂CO₃, that is discharged by the absorption unit 4, and the nutrients rich in nitrogen and/or phosphorus, are introduced and mixed in order to form a nutrient mixture that will constitute the nourishment of algal culture.

The nutrient mixture coming from the at least one mixing unit 5 is conveyed to the at least one photobioreactor (6) through a pipe along which an algal culture is inoculated.

Preferably, the gas flow q2 is directly introduced into the photobioreactor (6) by means of suitable systems that can maximize the contact surface-area between gas and liquid phase. Such systems can be of different typologies and in particular they can use suitable diffusers that allow to inject said gas in the form of fine bubbles. Moreover the gas can be injected in correspondence of a specific point of the pipe that constitutes the solar collector of the photobioreactor or, preferably, in different points of said photobioreactor. The direct inflow of a specific amount of gas in the photobioreactor 6 allows the absorption of additional amounts of CO₂ in the liquid and simultaneously increases the algal growth rate. Photosynthesis, which takes place in the photobioreactor (6), leads to a consumption of CO₂ and Na₂CO₃ in liquid phase as well as an alkalinization of the medium. It should be noted that that consumption of the main macro-nutrient in liquid phase, i.e. carbon, together with a too high alkalinization of the medium, may determine a significant slowdown of microalgal growth and in extreme cases its inhibition. Thus the direct inflow of CO₂ into the photobioreactor, advantageously avoids growth limiting phenomena due to macronutrient consumption and, at the same time, prevents a too high alkalinization of the solution.

In said at least one photobioreactor, algal biomass production takes place by photosynthesis.

Different typologies of photobioreactors may be employed but tubular ones should be preferred since they are less bulky and at the same time allow a more efficient exposition of their content to the light. A tubular photobioreactor consists of transparent pipes aligned to form an "array" of tubes that are typically made of plastic or glass. These "arrays" are the light collectors where the light is captured in order to allow the photosynthesis to take place. Preferably these tubes are less then 0.1 m in diameter since otherwise the light does not penetrate adequately in the less exposed zones of dense cultures. Thus in order to assure the suitable light intensity to algae, tubes being not too large in diameter should be preferred. However, larger diameters may be used when suitable regimes of turbulence of the fluid are employed in order to assure the movement of algae from the illuminated part of the tube to the dark one and vice-versa thus avoiding the algae to stay too long in the same point. This avoids photo-inhibition and photo-saturation phenomena to take place.

The tubes can be arranged in parallel or in series and then placed horizontally or vertically on the ground.

Several layers of horizontal pipes can be arranged like a fence in order to increase the number of tubes that can be placed in a given area. The tubes are preferably oriented north-south and the ground under the tubes is advantageously covered with white sheets of plastic in order to increase the albedo. In fact, a high albedo determines an increase of the total light received by the tubes.

Vertical tubes advantageously allow a more efficient use of available areas. This aspect is particularly advantageous when high amounts of carbon dioxide have to be captured since, in this case, the availability of land may be the main factor that limits the technical applicability of the process.

Thus according to the present invention the photobioreactors are preferably vertical tubes.

According to a preferred embodiment, algal culture flows upward in parallel through the tubes constituting the first array from bottom to top, then converges in a collector and, again, flows downward to be sent to the second array of tubes flowing again upward and so on for the other arrays. In this way, the flow occurs in parallel between different tubes, while the various arrays of tubes are arranged in series in order to ensure residence time that is sufficient to ensure algal growth and production of biomass.

An optional sedimentation of microalgae in tubes may be avoided by maintaining highly turbulent flow by means of a suitable mechanical or hydro-pneumatic pump (air-lift).

Advantageously, in order to avoid damage and inhibition of algal cells, the oxygen level should not preferably exceed about 400% of air saturation value. Since the oxygen produced by photosynthesis cannot be removed within the photobioreactor's tubes, advantageously algal culture is periodically sent to a degassing device where air is injected in order to strip dissolved oxygen of the algal culture. According to the present invention, the removal of oxygen is preferably carried out by means of venting systems or relief valves suitably placed all along the tubes.

Typically, a continuous tube length should not exceed 80 m (Molina Grima, E., Fernández, J., Acién Fernández, F. G., Chisti Y. Tubular photobioreactor design for algal cultures. Journal of Biotechnology, 92, 113-31 (2001)), but the choice of the exact length of the tube depends on several factors including biomass concentration, light intensity, flow rate, and dissolved oxygen concentration of the inlet solution. The degassing device, besides the oxygen excess, advantageously removes all the gas present into the fluid formed by seawater enriched in CO₂ and macro-nutrients.

Moreover, as the algal culture moves along photobioreactor's tubes, pH increases since carbon dioxide is consumed. Carbon consumption and pH increase may affect microalgae growth rate thus reducing the effectiveness of the entire process. In order to prevent these drawbacks, the gas flow q2, with a high CO₂ content, could be injected in suitable points placed at specific intervals along the tubes. This allows to advantageously prevent algae starving due to carbon dioxide consumption and too high value of pH.

Preferably, sensors are used to analyze whether concentration of macro-nutrients and pH value in the nutrient mixture exceed fall into suitable range. These sensors, if these values were not adequate, advantageously activate suitable dispensing devices that inject suitable amounts of nutrients.

During daylight hours photobioreactors are generally connected to a cooling and temperature control system. However, the temperature control device may be extremely advantageous also during night. In fact the nightly loss of biomass due to respiration can be reduced by lowering the temperature at night. Outdoor tubular photobioreactors may be effectively and inexpensively cooled using heat exchangers.

The outletting algal biomass from the at least one photobioreactor 6 is sent in the at least one biomass separation unit 7. Preferably, such a separation unit 7 is a settling tank or a filtering system or a centrifuge. Conveniently, said unit 7 is equipped with suitable sensors for the analysis of biomass concentration. Thus if the biomass concentration is suitably high, it is collected and sent to the harvesting section otherwise it is recycled at the top of the at least one photobioreactor 6. Finally the plant 1 of the present invention comprises at least a unit 8 for the extraction of bio-oil from the algal biomass separated in the at least one separation unit 7.

Bio-oil extraction can be carried out through different means but, according to the present invention, the use of mechanical presses is preferred in order to simplify plant operation and guarantee the economical feasibility. A liquid phase having a high triglycerides content, namely the bio-oil, and a solid, namely the residual solid cake result from the extraction unit 8.

Preferably, the plant 1 of the present invention comprises heat exchangers upstream the absorption unit 4, whereby the inlet gas is advantageously cooled. According to a preferred embodiment, the plant 1 of the present invention further comprises at least one drying unit 9 downstream the algal biomass separation unit 7. Preferably and advantageously, the heat needed for the evaporation of water from separated biomass can be provided by heat exchangers that are used to cool the inletting gas to the at least one absorption unit 4.

According to another embodiment, the plant 1 further includes at least one pipe for conveying the liquid, separated from biomass in the at least one separation unit 7, to the at least one absorption unit 4 for their recycling in the plant. In this way advantageous savings may be achieved both in terms of cost and in terms of process feasibility.

According to a preferred embodiment, the plant 1 of the present invention comprises:
i) a unit 2 for mixing the sea and/or fresh water with NaOH, this unit having:
   - at least a first inlet for feeding the sea and/or fresh water;
   - at least a second inlet for feeding NaOH; and
   - at least an outlet for the discharge of the absorbent liquid,
ii) a unit 3 for splitting the gas stream, deriving from the emission source, into two flows q1 and q2, this unit having:
   - at least a first inlet through which the entire gas stream, which comes from the emission source, flows;
   - at least a first outlet through which the gas flow q1 is discharged; and
   - at least a second outlet through which the gas flow q2 is discharged,
iii) an absorption unit 4 comprising at least a column where a solid support is packed, said column having:
   - at least a first inlet through which said absorbent liquid is fed;
   - at least a second inlet through which said gas flow q1, with high CO₂ content, is fed;
   - at least a first outlet from which said absorbent liquid enriched in CO₂ and Na₂CO₃, is discharged; and
   - at least a second outlet from which the gas depleted of CO₂ is discharged,
iv) a unit 5 for mixing said absorbent liquid enriched in CO₂ and Na₂CO₃ with a nutrients that are rich in nitrogen and phosphorus, said unit having:
   - at least a first inlet for feeding the absorbent liquid, enriched in CO₂ and Na₂CO₃, connected to said first outlet of the column of the absorption unit;
   - at least a second inlet for feeding the nutrient solution;
   - at least an outlet for discharging the obtained mixture,
v) a photobioreactor 6 for producing algal biomass, said photobioreactor having:
   - at least a first inlet for feeding the mixture coming from the at least one mixing unit, to which said first inlet port is connected by means of a tube along which an algal culture is injected; and
   - at least one or more inlets for feeding the gas flow q2 coming from unit 3 used for the splitting of the gas stream which comes from the emission source;
   - at least an outlet for the discharge of liquids and of algal biomass obtained in the photobioreactor;
vi) a unit 7 for separating biomass from liquids, said unit having:
   - at least an inlet for feeding the liquid and biomass outgoing the photobioreactor, connected to said photobioreactor outlet;
   - at least a first outlet for discharging the separated biomass;
   - at least a second outlet for discharging the separated liquid,
vii) a unit 8 for the extraction of bio-oil, said unit having:
   - at least an inlet for feeding the separated biomass, connected to the solid-liquid separation unit;
   - at least a first outlet for discharging the bio-oil obtained;
   - at least a second outlet for discharging the residual solid cake obtained.

A working Example of the present invention is herein below provided for illustrative and non limiting purposes.

### EXAMPLE. Implementing and working scheme of the plant for the production of bio-oil according to the present invention

A thermal power station was considered as source of carbon dioxide. This power station was located near the sea and was characterized by an electrical power generation of 1040 MW. In 2006, said thermal power station generated about 4749 GWh of energy and emitted about 3455 Mton of CO₂.

In this example, a plant for the production of bio-oil and its operating parameters were designed according to the present invention, considering the above mentioned thermal power station as CO₂ source. It was assumed to capture rate equal to 10% of the entire amount of fumes emitted by the power station. The choice of a rate of 10% derived from the necessity of compliance with the targets of CO₂ emission reduction as set forth for Italy in accordance with the subscribed agreements under the Kyoto Protocol. Therefore, in this regard, a reduction of 10% of CO₂ emissions with respect to the amounts emitted in 2006 for said thermal power station resulted to be satisfactory, even in view of the fact that the amounts emitted in 2006 resulted to be much higher than those emitted in 1990. Once specified the aspects underlying the choice of the fumes rate to capture, the plant was designed according to the process of the invention as shown in the attached Figure.

The fumes comprising a CO₂ content of 12 % vol/vol, were captured and then sent to absorption unit. As shown in Table 2, by assuming a thermal power station operating time of 7920 hours/year, the hourly emissions of CO₂ in 2006 were evaluated to be about 607 tons/year.

**Table 2. Evaluation of hourly emissions of CO₂ with respect to the plant operating time**

| **Parameter** | **Value** | **U.M.** |
|---|---|---|
| Annual operating hours | 7920 | h year⁻¹ |
| Operating days | 330 | days year⁻¹ |
| CO₂ emitted | 4807000 | t year⁻¹ |
| CO₂ emitted | 606.9 | t hour⁻¹ |
| Burned coal | 1726000 | t year⁻¹ |

In order to evaluate the total volumes and masses of emitted gas, an average gas composition shown in Table 3 was considered. The data shown in Table 3, were obtained by averaging the data found in the literature related to power stations. Moreover, the data shown in Table 3 were calculated by assuming the outlet chimney gas temperature to be about 100°C and pressure about 1 atm.

**Table 3. Average composition of fumes emitted by power stations and evaluation of the amounts to be treated**

| Gas | Volume %v/v | Molar fraction moles/molesₜₒₜ | Partial pressure atm | Molecular weight g mole⁻¹ | mass g | mass %wt/wt | Total mass in gas ton hour⁻¹ | Density normal conditions g L⁻¹ | Volume to be treated Nm³ hour⁻¹ |
|---|---|---|---|---|---|---|---|---|---|
| CO₂ | 15.1 | 0.151 | 0.151 | 44.01 | 21.7 | 0.222 | 606.900 | 1.962 | 309327 |
| H₂O | 7.9 | 0.079 | 0.079 | 18.00 | 4.6 | 0.048 | 129.860 | 0.803 | 161799 |
| O₂ | 6 | 0.06 | 0.06 | 32.00 | 6.3 | 0.064 | 175.337 | 1.427 | 122871 |
| N₂ | 71 | 0.71 | 0.71 | 28.01 | 64.9 | 0.666 | 1816.406 | 1.249 | 1454288 |
| Total | 100 | 1 | 1 | | 97.5 | 1.000 | 2728.503 | 1.332 | 2048307 |

Thus, by assuming to capture only the 10% of the emitted CO₂, the amounts of CO₂ to be treated are those shown in Table 4.

**Table 4.**

| Desired captation % | CO₂ mass flow to be treated ton hour⁻¹ | CO₂ volumetric flow to be treated Nm³ ora⁻¹ | Total flue-gas mass flow to be treated ton ora⁻¹ | Total flue-gas volumetric flow to be treated Nm³ ora⁻¹ |
|---|---|---|---|---|
| 10 | 60.69 | 30932.72 | 272.85 | 204830.66 |

According to the process of the invention, the fumes amounts shown in Table 4 were captured and, after cooling through heat exchangers, were sent to the absorption unit 4. In fact, this example related to the case where the gas flow q2 was close to zero. Said operating setting was selected in order to have indicative results of the minimal efficiency output of the plant, since an increase of the gas flow q2 inherently involved an increase of the efficiency output.

Within said absorption unit 4, sea water was employed. In the case of the present example, it was the Mediterranean Sea water, having an average composition so as to ensure a suitable concentration of micro-nutrients for the growth of most strains of microalgae used for bio-oil production.

The absorption columns were sized so as to guarantee a CO₂ removal efficiency from the inlet gas flow close to 99.99%. In packed columns, the solid support could be formed by Raschig rings, Pall rings, Berl saddles, or other similar typologies. Some characteristics of usable fillings, as well as characteristics of the packing, are shown in Table 5.

**Table 5.**

| **Packing characteristics** | **U.M.** | **Value** |
|---|---|---|
| Packing typology | | Raschig rings, Berg saddles, Pall rings, spiral rings, Lessing rings, or their combination |
| Material | | Ceramic or metallic |
| Packing constant | | 155.00 |
| Specific area | m² m⁻³ | 190.00 |
| Nominal diameter | m | 0.025 |
| Porosity | m³ m⁻³ | 0.7 |
| Critical surface tension | dyn cm⁻¹ | 40.00 |

Characteristics of fluid and of solid support that influence the design are shown in Table 1. It should be noted that the plant was supposed to be operating for 330 days/year for a total of 7920 hours/year. Calculations for sizing the absorption columns were performed according to the model proposed in the literature by Onda et al. (Onda, K., Takeuchi, H. and Okumoto, Y. Mass Transfer Coefficients Between Gas and Liquid Phases in Packed Columns. Journal of Chemical Engineering Japan, 1, 56-62 (1968)).

Sizing results, expressed in terms of columns characteristics and columns number that were needed for the removal of 99% of carbon dioxide from fumes by the power station, are shown in Table 6.

**Table 6, Chemical and physical properties of liquid and solid contacted in the absorption column**

| **Parameter** | **U.M.** | **Value** |
|---|---|---|
| Number of absorption columns | - | 69 |
| Material | - | concrete |
| Column diameter | m | 2.99 |
| Packed bed height | m | 7.76 |
| Overall area needing | m² | 1725 |

The overall volume of absorption unit was evaluated by considering a distance between the columns of about 4 m. Table 7 shows the characteristics of inletting and outletting flows from columns, the flows of water to be used as well as the flows of each component present in the flows.

**Table 7**

| | **Liquid Inlet** | **Gas Inlet** | **Gas outlet** | **Gas outlet** |
|---|---|---|---|---|
| Physical properties | | | | |
| Temperature (°C) | 21 | 20 | 21 | 21 |
| Pressure (bar) | 1.01 | 1.01 | 1.01 | 1.01 |
| Density (g/L) | 996.16 | 1.35 | 1.25 | 898.24 |

| Chemical properties (t/h) | | | | |
|---|---|---|---|---|
| CO₂ | 0.0 | 60.6 | 0.6 | 60.0 |
| N₂ | 0.0 | 181.7 | 181.7 | 0.0 |
| O₂ | 0.0 | 17.5 | 17.5 | 0.0 |
| H₂O | 300000.0 | 0.0 | 0.0 | 300000.0 |
| TOTAL (t/h) | 300000.0 | 272.9 | 212.9 | 300060.0 |
| TOTAL (m³/h) | 301155.7 | 202315.9 | 170106.8 | 334054.7 |

From Table 7, a high amount of water to be used (~ 300000 m³ hour⁻¹) were observed for promoting the desired CO₂ mass transfer. Although high, these amounts of water were comparable to those used by the power station for turbine steam and machines cooling, that in 2006 was about 1087×10⁶ m³ year⁻¹, which, taking into account an operating time of 7920 ore year⁻¹, resulted in about 140000 m³ hour⁻¹. Moreover, as already specified in the detailed description of the invention, it was possible for these flows to be pumped from the sea only during the start up of the plant, since, once the stationary regime was reached, most of the water used could be provided by recycling of the nutrient mixture if the micro-nutrients were not totally consumed during the algal growth cycles. Therefore at stationary regime, the seawater flow to be pumped was only the amount necessary to integrate losses in the recirculation device. Moreover in case the unit 2 for mixing seawater with NaOH was used, the consequent chemical reaction taking place between CO₂ and NaOH in liquid phase, caused a significant reduction of the amount of water needed to carry out absorption of the same amounts of CO₂.

In Table 8 shows the concentration of carbon dioxide dissolved in liquid phase. It is worth remarking that CO₂ was the main nutrient for algal growth within photobioreactors.

**Tabella 8. Concentration of carbon dioxide of the outletting liquid from the absorption column and thus at the inlet of photoreactor**

| | Flow | CO₂ concentration |
|---|---|---|
| | t hour⁻¹ | g L⁻¹ |
| Inlet | | |
| Liquid IN | 300000.0 | 0 |
| Gas IN | 272.9 | 0.29942 |

| Outlet | | |
|---|---|---|
| Liquid OUT | 300060.0 | 0.179526 |
| Gas OUT | 212.9 | 0.003561 |

Once evaluated the seawater flow to be sent to the absorption unit, it was possible to calculate the number of pumps to be used and their operating conditions. As reported in Tables above, the seawater flow to be sent to the columns was approximately 300000 m³ hour⁻¹. By taking into account that the liquid was sent to the top of absorption columns, that were 7 m in height, and assuming the pressure losses in piping of about 3 m of water, the prevalence that had to be assured by pumps was of about 10 m, the term "prevalence" meaning the hydraulic jump or hydraulic head which can be expressed in meters of water column (head) or in atm (pressure).

Characteristics and number of pumps to be used for pumping the seawater to the absorbers are reported in Table 9.

**Table 9. Sizing results of the pumping section**

| **Parameter** | **U.M.** | **Value** |
|---|---|---|
| Typology | - | Centrifugal |
| Material | - | Steel |
| Power of each pump | kW | 299 |
| Pump head | M | 10 |
| Total flow | M³ ora⁻¹ | 300000 |
| Number of pumps | - | 40 |
| Flow of each pump | M³ ora⁻¹ | 7500 |
| Utilizatin factor at regime | Ore ₚₒₘₚₑ Oreₜₒₜₐₗᵢ⁻¹ | 0.5 |
| Totale electric energy consumed | MWh anno⁻¹ | 118484.6 |

Herein below, the flow of wastewater to be added in order to ensure the suitable concentrations of macro-nutrients (nitrogen and phosphorus) for algal growth, were evaluated. By comparing the concentration of macronutrient typically required for algal growth with those resulting from seawater chemical composition, it was extrapolated that only phosphorous was not present in suitable concentration. Generic stoichiometric schemes found in the literature and from plant practice, showed that for each 1.7 kg of CO₂, the algae employed for growing about 40 g of nitrogen and 4 g of phosphorus respectively. Thus, by taking into account that concentration of carbon dioxide in the outletting seawater from the columns was of 0,179526 g L⁻¹, about 0,004224141 g L⁻¹ of N and 0,000422414 g L⁻¹ of P were respectively needed. The comparison between nitrogen and phosphorus concentration in the outletting liquid from the columns and their minimum required value for assuring algal growth is shown in Table 10.

**Table 10. Concentration of macronutrients and comparison with minimum values**

| Compound | Concentration in the outlet liquid from the absorption unit g/L | Minimum required concentration g/L |
|---|---|---|
| N total (NH₄+NO₃) | 0.0155 | 0.00422 |
| P total (H₂PO₄) | 0.000088 | 0.000422414 |

From Table 6, it was observed that, while nitrogen concentration was sufficient to sustain algal growth, phosphorus concentration was lower than the minimum concentration required. It was therefore necessary to integrate the phosphorus and possibly increase concentrations of nitrogen, by mixing seawater from the columns with small amounts of wastewater that have been treated but not undergone to denitrification and dephosphatization processes.

Since concentrations in the effluents was a known parameter, the sizing problem was reduced to the evaluation of the flow of treated wastewater that has to be added in order to obtain the required minimum proportions between nitrogen and carbon dioxide and between phosphorus and carbon dioxide. The results of the calculation of the flow of wastewater to be added is shown in Table 11 below where are also typical values of concentration of nitrogen and phosphorus in the outlet wastewater from treatment plants where no denitrification or dephosphatization processes were carried out. Table 11. Evaluation of wastewater flows

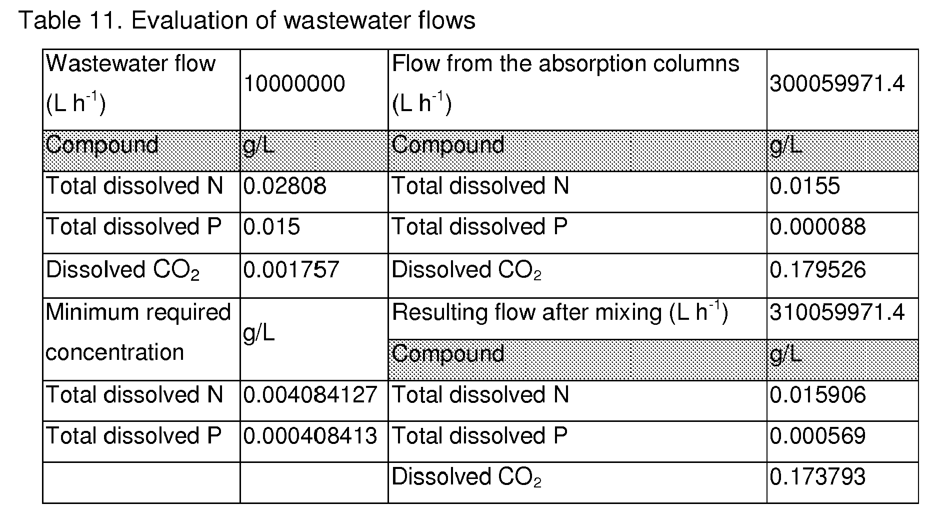

From Table 11, it could be observed that an amount of wastewater of 10000 m³ h⁻¹ (only 3.3% of total flow coming from the columns) was sufficient to ensure suitable proportions of carbon, nitrogen and phosphorus desired. Since the feeding of the two flows to be mixed is constant, mixing could be carried out through simply conveying the two flows in the same pipe. Thus no specific operating unit had to be implemented in order to carry out this mixing step.

The mixture obtained after absorption of carbon dioxide and after mixing with wastewaters, contained all the nutrients that ensure microalgal growth and propagation. Finally, in order to ensure the photosynthesis, after microalgae inoculation, the algal culture was sent to the tubular photobioreactors where the suitable light energy was provided.

In this example, a specific photobioreactor typology already available on the market was considered. In Table 12, the characteristic of each photobioreactors' module considered in this example are summarized.

**Table 12. Properties of each photobioreactors' module**

| Parameter | Value | Unit of measurement |
|---|---|---|
| **Geometric properties** | | |
| Max height of each tube | 12.00 | m |
| Total length of tubes | 30000.00 | m |
| Tube diameter | 0.90 | m |
| Effective section area of tube | 0.64 | m² |
| Total effective volume of tubes | 19085.18 | m³ |
| N ° of tubes per module | 2500.00 | - |

| **Energy consumption** | | |
|---|---|---|
| For each tube | 0.75 | kW tube⁻¹ |
| Overall energy consumption | 1875 | kW |

| **Photobioreactor productivity** | | |
|---|---|---|
| Daily | 100 | t_{dry biomass} . day⁻¹ |
| Annually | 33000 | t_{dry biomass} . year⁻¹ |

| **Fixed carbon dioxide** | | |
|---|---|---|
| Daily | 200000 | kg . day⁻¹ |
| Annually | 66000 | t . year⁻¹ |

| **Module cost (only photobioreactors or related)** | | |
|---|---|---|
| Module cost | 10000000 | Euro |
| Module cost | 10 | MEuro |

| **Operating cost (only photobioreactor or related, energy include)** | | |
|---|---|---|
| For unit mass of biomass produced (10 cents/kg) | 100 | Euro t_{dry biomass}⁻¹ |
| Overall operating costs | 3300000 | Euro anno⁻¹ |

The sizing problem of photobioreactors thus led to the calculation of the number of modules necessary to guarantee the treatment of the whole CO₂ that had been captured. For performing the sizing, it was taken into account that carbon dioxide amounts to be captured were those dissolved in the outletting liquid from the absorption unit and the mixing unit.

Photobioreactors were assumed to operate for 10 hours/day and for 330 days/year (daylight hours). Characteristics of the nutrient mixture for microalgae are shown in Table 14.

**Table 14 Operating parameters of photobioreactors and flows to be treated**

| | | | |
|---|---|---|---|
| Operating time of absorbers (h day⁻¹) | 24 | | |
| Operating time of photobioreactors (h giorno⁻¹) | 10 | | |
| Flow fed to the PBRs only during daylight hours (L h⁻¹) | 744143931 | | |

| **Nutrient** | **Concentration g L⁻¹** | **Load kg h⁻¹** | **Load kg day⁻¹** |
|---|---|---|---|
| Total N | 0.015906 | 11836 | 118362 |
| Total P | 0.000569 | 423 | 4234 |
| Total dissolved CO₂ | 0.173793 | 129327 | 1293267 |

In order to establish the number of photobioreactors' modules needed, it was therefore sufficient to divide the total load of CO₂ to be captured, by the load of CO₂ that each photobioreactor can capture. The only sizing validation criteria to be performed was the one concerning the fluid velocity within the tubes that should be at least 8 cm s⁻¹. This value, in fact was such as to assure a suitable turbulent flow that, in turn, could enable the exposition of microalgae cells to light-dark cycles that optimize photosynthesis avoiding photo-saturation and photo-limitation phenomena. Such validation was performed by taking into account that the overall liquid flow, to be distributed over the different modules, was passed through an array of 500 tubes for each module (square modules having 2500 tubes) characterized by the section already defined in Table 15.

**Table 15. Sizing results and evaluation of fluid velocity in the tubes**

| **Parameter** | **Value** |
|---|---|
| Number of modules (-) | 6 |
| Flow fed at each module (m³ h⁻¹) | 124024 |
| Flow fed at each module (m³ s⁻¹) | 34 |
| Number of tubes of the first array of the module (-) | 500 |
| Fluid velocity within tubes (cm sec⁻¹) | 11 |

From Table 15, it can be noted that the resulting fluid velocity value in photobioreactors was such as to satisfy the above defined validation criterion and therefore the sizing results can be considered valid. According to design, the plant thus consisted of 6 modules of photobioreactors whose characteristics are described in Table 14. The characteristics of the entire plant are summarized in Table 16.

**Table 16. Properties of the whole plant**

| **Parameter** | **Value** | **U.M.** |
|---|---|---|
| Geometrical properties | | |
| Max height of each tube | 12.00 | m |
| Total length of tubes | 1080000.00 | m |
| Tube diameter | 0.90 | m |
| Effective section area of tube | 0.64 | m² |
| Total effective volume of tubes | 687066.31 | m³ |
| N° of tubes per module | 15000.00 | - |

| Energy consumption | | |
|---|---|---|
| For each tube | 0.75 | kW tube⁻¹ |
| Overall energy consumption | 11250 | kW |

| Plant productivity | | |
|---|---|---|
| Daily | 600 | t_{dry biomass} . day⁻¹ |
| Annual | 198000 | ^{t}_{dry biomass} . year⁻¹ |

| Fixed carbon dioxide | | |
|---|---|---|
| Fixed carbon dioxide | 1200000 | kg . day⁻¹ |
| Fixed carbon dioxide | 396000 | t . year⁻¹ |

| Plant cost (only photobioreactors or related) | | |
|---|---|---|
| Plant cost | 60000000 | Euro |
| Plant cost | 60 | MEuro |

| Operating cost (only photobioreactor or related, energy included) | | |
|---|---|---|
| For unit mass of biomass produced (10 cents/kg) | 100 | Euro t_{dry biomass}⁻¹ |
| Overall operating costs | 19800000 | Euro year⁻¹ |

According to the present example, the process thus implemented through the designed plant led to the production of a "slurry" characterized by a high humidity level and containing the algal biomass. According to the flow-sheet shown in Figure, the following step of the invention was the separation of solid (biomass) from liquid, being this step performable by means of settling tanks or hydrocyclones or filter presses. At the outlet the solid was sent to suitable dryers for a further removal of residual water. The liquid obtained was recirculated at the top of the absorption unit as shown in Figure.

From the dehydration and drying steps, a dried biomass in form of powders or lumps green coloured was obtained.

This product was sent to a pressing unit where the oil was extracted from microalgae. The oil extracted, which, averagely, was characterized by a high calorific value (~30000 MJ ton⁻¹), was called bio-oil. As mentioned in the detailed description of the invention, the algal cells may show an oil content ranging from 30% to 80% in weight, depending both upon the alga strain used and on the operating conditions. In what follows, it was assumed that oil content of microalgae is of 40%w/w. Thus, by pressing the dried biomass, the bio-oil obtained amounted to the 40% of total biomass weight while the remaining 60% consisted of a residual solid cake or press cake. On the base of this assumption, the amounts of bio-oil and press-cake shown in Table 17, can be produced.

**Tabella 17**

| Parameter | Value | U.M. |
|---|---|---|
| Algae oil content | 0.40 | kgₒᵢₗ . kg_{dry_bimass}⁻¹ |
| Bio-oil production | 79200 | t. year⁻¹ |
| Press-cake (proteins, carbohydrates, etc.) | 118800 | t. year⁻¹ |
| CO₂ fixed | 480700 | t. year⁻¹ |

Bio-oil could be further processed in-situ through transesterification process for producing biodiesel to sell in the fuel market. Otherwise the bio-oil could be sold as such, like crude oil, to be further refined in different plants. It is worth noting that the yield of transesterification process was about 80% by weight, thus from 79200 tons/year of bio-oil produced, about 63360 ton/year of biodiesel could be obtained. Such amounts could satisfy a significant percentage of the biodiesel needed of a whole Italian region for the transportation sector.

As far as the other flow of material produced by pressing is concerned, namely the residual solid cake, different exploitations were possible depending upon its compositional characteristics. In fact, some applications, have shown that, from specific algal strains it was possible to vitamins or antioxidants such as carotene and astaxanthin.

The residual solid cake obtained from algae pressing could be even used in the pharmaceutical sector since it can contain, for example, sterols that are used for hormones synthesis. Finally, some cyano-bacteria can be advantageously employed in the biomedical field for the production of antimicrobial, antiviral and anticancer.

Even if, for reasons related to the size of market demand or for their compositional characteristics, the cake is not marketable, it could be recycled in the power station itself. The cake, in fact, has a good residual calorific value, and once packed in the form of "croquettes" they constitute the so-called green coal that may be burned in the combustion chamber along with the coal from the mines. Even in this case a significant energy savings may be achieved.

From the detailed description and from the example above reported, it is apparent that through the process of the present invention as well as through the implementation of the related plant, significant advantages may be achieved. In particular, the process allows the production of bio-oil and at the same time the removal of pollutants gases (in particular CO₂) from punctual emission sources.

In fact, the step of pre-treatment of pollutant gas, which is carried out by contacting the gas having seawater during the contemporaneous passage through a suitable support solid that allows obtaining the transfer of CO₂ from gas to liquid, ensures the achievement of CO₂ sequestration so that, huge amounts of CO₂ are available for photosynthesis, while the outlet gas that is discharged in the atmosphere is advantageously depleted of CO₂.

Moreover the use of algae determines important energy savings and decreases environmental impacts since agricultural lands are not needed for their cultivation. According to the invention, algae are grown by using nutrients such as CO₂ from flue-gas as well as nitrogen and phosphorus from wastewaters. Therefore it is possible to produce a costless nutrient mixture thus reducing at the same time the diffusion in the environment of pollutants that are instead used as nutrients in the process.

Moreover, once the plant operates at a stationary regime, economical feasibility of the process and its convenience are ensured by several material recirculation steps.

## Claims

1. Process for the production of bio-oil comprising the steps of:
a1) providing a gas having high CO₂ content;
a2) providing sea and/or fresh water for the production of the culture medium;
b1) splitting the stream of said gas into two flows q1 and q2;
b2) passing the gas flow q1, having high CO₂ content, through a solid support;
b4) passing the sea and/or fresh water through the same solid support of step b2) in order to subtract CO₂ from said gas, said step b4) being simultaneously performed with step b2);
c) adding nitrogen-rich and/or phosphorus-rich nutrients to the sea and/or fresh water, previously enriched in CO₂, to form a nutrient mixture;
d1) contacting said nutrient mixture with an algal culture;
d2) contacting said nutrient mixture and said algal culture with the gas flow q2;
e) exposing said algal culture, contacted with the nutrient mixture and with the gas flow q2, to a light source capable to promote photosynthesis, thus obtaining the formation of algal biomass;
f) separating the obtained algal biomass; and
g) extracting bio-oil from the algal biomass.

2. The process according to claim 1, further comprising a step b3), to be carried out after step b2) and before step b4), wherein said sea and/or fresh water is mixed with NaOH.

3. The process according to claim 1 or 2, wherein said solid support is made of ceramic or metal material.

4. The process according to any one of claims 1-3, wherein the algal culture consists of microalgae.

5. The process according to any one of claims 1-4, wherein the gas phase with high CO₂ content of step b2) passes through the solid support in opposite direction to the one along which the sea and/or fresh water of step b4) passes through the same support.

6. The process according to any one of claims 1-5, wherein the gas provided in step a1) comprises exhaust gases from thermal power stations or industrial plants or other sources of CO₂.

7. The process according to any one of claims 1-6, wherein nutrients of step c) are added in the form of wastewater.

8. The process according to claim 7, wherein said wastewater are purified, not denitrified, not dephosphorized, and optionally pasteurized.

9. The process according to any one of claims 1-8, further comprising a step h) wherein algal biomass is dried after the step f).

10. The process according to claim 9, wherein the gas is cooled before being provided in step a1) and the heat subtracted to the gas is used for drying algal biomass in step h).

11. A plant (1) for the production of bio-oil, including:
- at least one unit (3) for splitting the gas stream into two gas flows q1 and q2;
- at least one absorption unit (4) for the transfer of CO₂ from gas flow q1, having high content of CO₂ in sea and/or fresh water, this unit including at least one column wherein a solid support is packed;
- at least one mixing unit (5) of said sea and/or fresh water enriched in CO₂ and nutrients rich in nitrogen and phosphorus;
- at least a photobioreactor (6) for the production of algal biomass, wherein an algal culture contacts the outletting mixture from said mixing unit (5) and the gas flow q2 having a high CO₂ content;
- at least one unit (7) for separating the biomass from the fluid outgoing from the at least one bioreactor (6); and
- at least one unit (8) for extracting the bio-oil from the separated biomass.

12. The plant (1) according to claim 11, wherein the at least one absorption unit (4) for transferring of CO₂, includes at least one column, in which, the gas flow passes along a direction that is opposite to the direction along which sea and/or fresh water passes through the same column.

13. The plant (1) according to claim 11 or 12 further comprising at least one mixing unit (2) wherein sea and/or fresh water and NaOH are fed and then mixed for producing a mixture called absorbent liquid.

14. The plant (1) according to anyone of claims 11-13, wherein:
i) a unit (2) for mixing the sea and/or fresh water with NaOH, this unit having:
- at least a first inlet for feeding the sea and/or fresh water;
- at least a second inlet for feeding NaOH; and
- at least an outlet for the discharge of the absorbent liquid,
ii) a unit (3) for splitting the gas stream, deriving from the emission source, into two flows q1 and q2, this unit having:
- at least a first inlet through which the entire gas stream, which comes from the emission source, flows;
- at least a first outlet through which the gas flow q1 is discharged; and
- at least a second outlet through which the gas flow q2 is discharged,
iii) an absorption unit (4) comprising at least a column where a solid support is packed, said column having:
- at least a first inlet through which said absorbent liquid is fed;
- at least a second inlet through which said gas flow q1, with high CO₂ content, is fed;
- at least a first outlet from which said absorbent liquid enriched in CO₂ and Na₂CO₃, is discharged; and
- at least a second outlet from which the gas depleted of CO₂ is discharged,
iv) a unit (5) for mixing of said absorbent liquid enriched in CO₂ and Na₂CO₃ with a nutrients that are rich in nitrogen and phosphorus, said unit having:
- at least a first inlet for feeding the absorbent liquid, enriched in CO₂ and Na₂CO₃, connected to said first outlet of the column of the absorption unit;
- at least a second inlet for feeding the nutrient solution;
- at least an outlet for discharging the obtained mixture,
v) a photobioreactor (6) for producing algal biomass, said photobioreactor having:
- at least a first inlet for feeding the mixture coming from the at least one mixing unit, to which said first inlet port is connected by means of a tube along which an algal culture is injected; and
- at least one or more inlets for feeding the gas flow q2 coming from unit 3 used for the splitting of the gas stream which comes from the emission source;
- at least an outlet for the discharge of liquids and of algal biomass obtained in the photobioreactor;
vi) a unit (7) for separating biomass from liquids, said unit having:
- at least an inlet for feeding the liquid and biomass outgoing the photobioreactor, connected to said photobioreactor outlet;
- at least a first outlet for discharging the separated biomass;
- at least a second outlet for discharging the separated liquid,
vii) a unit (8) for the extraction of bio-oil, said unit having:
- at least an inlet for feeding the separated biomass, connected to the solid-liquid separation unit;
- at least a first outlet for discharging the bio-oil obtained;
- at least a second outlet for discharging the residual solid cake obtained.

15. The plant (1) according to any one of claims 11-14, wherein the inletting gas to the at least one absorption unit (2) is cooled upstream the latter by means of heat exchangers, the plant further comprising at least one drying unit (9), downstream the at least one algal biomass separation unit (5), such that the heat subtracted by heat exchangers is supplied to the at least one drying unit (9) in order to dry the algal biomass.
